# EUROPÄISCHE PATENTSCHRIFT

(11) **EP 0 675 872 B1**
(45) Veröffentlichungstag und Bekanntmachung des Hinweises auf die Patenterteilung: **30.07.1997**
(21) Anmeldenummer: 94903761.8
(22) Anmeldetag: 13.12.1993
(51) Int. Cl.: C07C 219/06, C07C 219/08, C07F 9/113, D06M 13/372

(54) **QUATERNIERTE FETTSÄURE-TRIETHANOLAMINESTER-SALZE**
QUATERNARY FATTY-ACID TRIETHANOLAMINE ESTER SALTS
SELS QUATERNAIRES D'ESTERS DE TRIETHANOLAMINE D'ACIDE GRAS

(30) Priorität: 22.12.1992 DE 4243547
(43) Veröffentlichungstag der Anmeldung: 11.10.1995
(73) Patentinhaber: Henkel Kommanditgesellschaft auf Aktien, 40191 Düsseldorf (DE)
(72) Erfinder: BIGORRA LLOSAS, Joaquim, E-08203 Sabadell (ES); LOPEZ GOMEZ, Maria Dolors, E-08015 Barcelona (ES); SOLER CODINA, Antoni, E-08221 Teressa (ES)
(86) Internationale Anmeldenummer: EP9303517
(87) Internationale Veröffentlichungsnummer: WO9414756

(56) Entgegenhaltungen:
- EP-A- 0 075 168
- EP-A- 0 131 865
- WO-A-91/01295

## Beschreibung

### Gebiet der Erfindung

Die Erfindung betrifft quaternierte Fettsäure-triethanolaminester-Salze, ein Verfahren zu ihrer Herstellung, bei dem man Fettsäuren mit Triethanolamin oder Triethanolaminethoxylaten verestert und anschließend in Gegenwart von langkettigen Phosphorsäureestern mit Ethylenoxid quaterniert, Spinnfaserpräparationen, die diese Salze enthalten sowie die Verwendung der Salze zur Herstellung von Spinnfaserpräparationen.

### Stand der Technik

Synthetische Fasern werden nach dem Filamentbildungsprozeß mit Präparationsmitteln versehen, die für ihre Weiterverarbeitung unentbehrlich sind. Diese sogenannten "Spinnfaserpräparationen" setzen die Reibung sowohl zwischen den Filamenten untereinander, als auch zwischen den Filamenten und den Führungselementen der Spinnmaschinen herab [**Ullmanns Encyclopaedie der technischen Chemie, Bd. 23, S.7-9, Verlag Chemie, Weinheim, 1983)**].

Aus der Europäischen Patentanmeldung **EP-A 0 131 865** sind oxalkylierte quaternäre Ammoniumverbindungen bekannt, die als reibungsherabsetzende Mittel für die Herstellung von Faserpräparationen beschrieben werden.

Neben der Glätte und dem Fadenschluß werden von den Faserherstellern in der Regel noch weitere Anforderungen an Faserpräparationen gestellt, von denen die Verminderung der elektrostatischen Aufladung, die Avivage der Fasern, die Verträglichkeit mit Farbstoffen sowie die gute biologische Abbaubarkeit der Präparationen die wichtigsten sind. Neben den genannten Anforderungen müssen Faserpräparationen temperaturbeständig und nicht korrosiv, von der Faser leicht zu entfernen und physiologisch unbedenklich sein.

Wie leicht einzusehen ist, kann dieses umfangreiche Anforderungsprofil kaum durch eine einzelne Substanz abgedeckt werden. Moderne Faserpräparationen stellen daher in der Regel Gemische verschiedener Stoffe dar, deren Zusammenspiel erst den gewünschten Effekt bei der Faserherstellung liefert. Typische Beispiele für Inhaltsstoffe von Spinnfaserpräparationen sind synthetische Esteröle, Sorbitanester, Silicone, Polyether, Phosphorsäureester, quaternierte Fettamine, Fettsäurealkanolamine, ethoxylierte Fettsäuren sowie Blockpolymere des Ethylen- und Propylenoxids ("Pluronic-Typen") und dergleichen.

Die Aufgabe der Erfindung bestand nun darin, neue Einzelstoffe für die Herstellung von Spinnfaserpräparationen zur Verfügung zu stellen, die das geschilderte Anforderungsprofil möglichst vollständig erfüllen.

### Beschreibung der Erfindung

Gegenstand der Erfindung sind quaternierte Fettsäure-triethanolaminester-Salze der Formel (I), 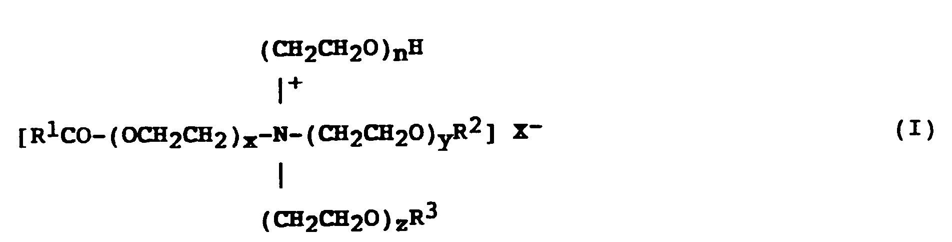 in der R¹CO für einen Acylrest mit 6 bis 22 Kohlenstoffatomen, R² und R³ unabhängig voneinander für Wasserstoff oder R¹CO, n für Zahlen von 1 bis 10, x, y und z für 1 oder Zahlen von 2 bis 30 und X für ein langkettiges Alkyl- und/oder Dialkylphosphat steht.

Überraschenderweise wurde gefunden, daß die erfindungsgemäßen Salze sowohl die Reibung zwischen synthetischen Faserfilamenten, als auch die elektrostatische Aufladung herabsetzen. Sie besitzen ferner gegenüber Fasern avivierende Eigenschaften, sind hochtemperaturstabil, biologisch leicht abbaubar und physiologisch unbedenklich. Ein besonderer Vorteil besteht darin, daß die erfindungsgemäßen Salze mit Farbstoffen verträglich sind und ein "Ausbluten" verhindern. Da die Produkte in Wasser gut löslich bzw. dispergierbar sind, können sie ferner leicht auf die Faser aufgebracht und wieder entfernt werden.

Ein weiterer Gegenstand der Erfindung betrifft ein Verfahren zur Herstellung von quaternierten Fettsäuretriethanolaminester-Salzen der Formel **(I)**, 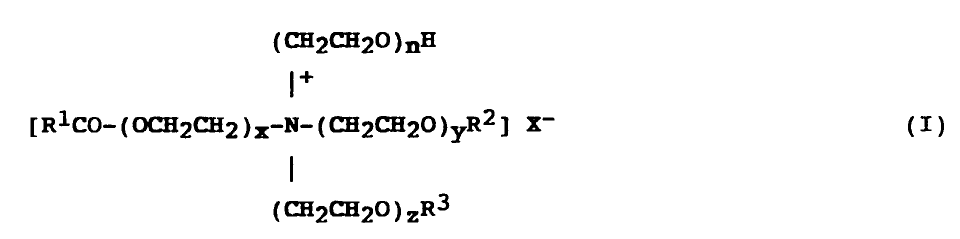 in der R¹CO für einen Acylrest mit 6 bis 22 Kohlenstoffatomen, R² und R³ unabhängig voneinander für Wasserstoff oder R¹CO, n für Zahlen von 1 bis 10, x, y und z für 1 oder Zahlen von 2 bis 30 und X für ein langkettiges Alkyl- und/oder Dialkylphosphat steht, bei dem man Fettsäuren mit Triethanolamin oder Triethanolaminethoxylaten verestert und anschließend in Gegenwart von langkettigen Phosphorsäure-mono/di-estern mit Ethylenoxid quaterniert.

### Ausgangsstoffe:

**Fettsäuren**, die im Sinne des erfindungsgemäßen Verfahrens mit Triethanolamin oder Triethanolaminethoxylaten partiell oder vollständig verestert werden, folgen der Formel (II),

**R**^{**4**}**CO-OH (II)**

in der R⁴CO für einen aliphatischen, gesättigten oder ungesättigten Acylrest mit 6 bis 22 Kohlenstoffatomen steht. Typische Beispiele sind Capronsäure, Caprylsäure, Caprinsäure, Laurinsäure, Myristinsäure, Palmitinsäure, Palmoleinsäure, Stearinsäure, Isostearinsäure, Ölsäure, Elaidinsäure, Petroselinsäure, Linolsäure, Linolensäure, Arachinsäure, Gadoleinsäure, Behensäure und Erucasäure sowie deren technische Gemische, wie sie beispielsweise bei der Druckspaltung von pflanzlichen Fetten und Ölen anfallen. Quaternierte Fettsäure-triethanolaminester-Salze der Formel (I) mit besonders vorteilhaften Eigenschaften werden erhalten, wenn man als Fettsäuren gehärtete, partiell gehärtete oder ungehärtete C_{16/18}-Talgfettsäuren oder technische Gemische mit hohem Elaidinsäuregehalt einsetzt.

**Triethanolamin** und **Triethanolaminethoxylate** stellen bekannte Stoffe dar, die nach den einschlägigen Methoden der präparativen organischen Chemie erhalten werden können. Sie folgen der Formel (III), 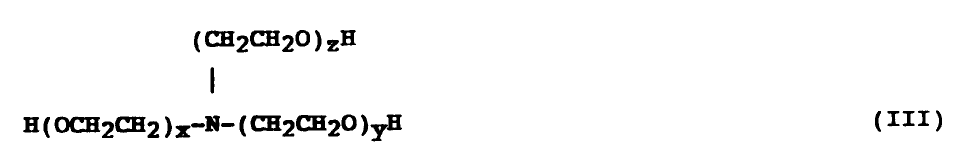 in der x, y und z für 1 oder Zahlen von 2 bis 30 stehen können.

Wie aus der Formel ersichtlich ist, bedeutet der Fall x = y = z = 1 das nicht-ethoxylierte Triethanolamin, welches als Ausgangssubstanz besonders bevorzugt ist. Steht mindestens einer der Indices für Zahlen von 2 bis 30, liegt ein ethoxyliertes Triethanolamin vor. Da Triethanolaminethoxylate üblicherweise durch statistische Umsetzung von Triethanolamin mit Ethylenoxid hergestellt werden, stellt der Ethoxylierungsgrad E, den man als (x+y+z) - 3 definieren kann, nur einen Durchschnittswert dar. Als Ausgangsstoffe bevorzugt sind weiterhin Triethanolaminethoxylate mit einem Ethoxylierungsgrad von 2 bis 7, d.h. Umsetzungsprodukte von Triethanolamin mit durchschnittlich 2 bis 7 Mol Ethylenoxid.

Üblicherweise werden die Fettsäuren und das Triethanolamin bzw. die Triethanolethoxylate im molaren Verhältnis von 1 : 1,0 bis 1 : 3,0 eingesetzt. Als besonders vorteilhaft haben sich quarternierte Fettsäuretriethanolaminester-Salze erwiesen, in denen das Fettsäure : Amin-Verhältnis einen Wert von 1,2 bis 2,2 aufweist. Die bevorzugten Salze stellen also Gemische von quaternierten Mono-, Di- und Trifettsäureestern, vorzugsweise mit einem hohen Gehalt an Monoestern dar.

### Veresterung

Die Veresterung kann in an sich bekannter Weise durchgeführt werden, wie sie beispielsweise in der Internationalen Patentanmeldung **WO 91/1295** (Henkel) beschrieben worden ist. Vorteilhafterweise erfolgt die Veresterung bei Temperaturen von 120 bis 220°C und Drücken von 0,01 bis 1 bar. Es sei an dieser Stelle darauf hingewiesen, daß die genannte Schrift keinen Hinweis auf quaternierte Fettsäure-triethanolaminester in Form ihrer langkettigen Alkyl- und/oder Dialkylphosphat-Salze oder die Verwendung dieser oder ähnlicher Stoffe für die Faserpräparation enthält.

### Quaternierung

Im Sinne des erfindungsgemäßen Verfahrens findet die Quaternierung in Gegenwart eines langkettigen Phosphorsäuremono/diesters der Formel **(IV)** statt, 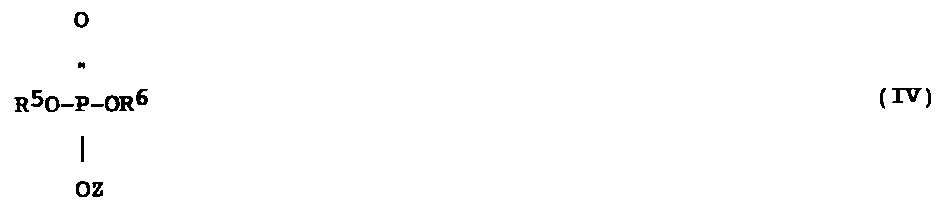 in der R⁵ für einen Alkyl- und/oder Alkenylrest mit 4 bis 18 Kohlenstoffatomen oder einen Rest R⁷(OCH₂CH₂)ₘ, R⁶ für Wasserstoff, einen Alkyl- und/oder Alkenylrest mit 4 bis 18 Kohlenstoffatomen oder einen Rest H(OCH₂CH₂)ₘ, R⁷ für einen Alkyl- und/oder Alkenylrest mit 4 bis 18 Kohlenstoffatomen, m für Zahlen von 1 bis 8 und Z für Wasserstoff oder ein Alkalimetall steht.

Vorzugsweise erfolgt die Quaternierung in Gegenwart von Phosphorsäuremono/diestern der Formel **(IV)**, in der R⁵ für einen Alkylrest mit 8 bis 18, insbesondere 12 bis 18 Kohlenstoffatomen und R⁶ für Wasserstoff oder R⁴ steht. Aus anwendungstechnischer Sicht haben sich Phosphorsäuremono- und/oder -di-laurylester als besonders vorteilhaft erwiesen.

Üblicherweise werden die Veresterungsprodukte von Fettsäure und Triethanolamin bzw. Triethanolaminethoxylat und die Phosphorsäureester im molaren Verhältnis von 1 : 0,5 bis 1 : 1,5, vorzugsweise 1 : 0,9 bis 1 : 1,2 eingesetzt. Dem Phosphorsäureester fällt dabei sowohl die Aufgabe zu, den Stickstoff zu protonieren und somit als Katalysator für die Ethoxylierung zu fungieren, als auch das Gegenion der quartären Ammoniumverbindung zu liefern.

Die Veresterungsprodukte von Fettsäure und Triethanolamin bzw. Triethanolaminethoxylat und das Ethylenoxid können im molaren Verhältnis von 1 : 1 bis 1 : 10, vorzugsweise 1 : 2 bis 1 : 5 eingesetzt werden. Die Quaternierung kann lösungsmittelfrei aber auch in Gegenwart von Wasser oder einem wäßrigen kurzkettigen Alkohol bei Temperaturen von 50 bis 170, vorzugsweise 90 bis 120°C und 0,5 bis 10, vorzugsweise 1 bis 4 bar durchgeführt werden.

### Gewerbliche Anwendbarkeit

Die erfindungsgemäßen quaternierten Fettsäure-triethanolaminester-Salze vermindern die Reibung zwischen Faserfilamenten und setzen die elektrostatische Aufladung herab. Sie besitzen avivierende Eigenschaften, sind gegenüber Farbstoffen inert, temperaturstabil und biologisch leicht abbaubar.

Ein weiterer Gegenstand betrifft daher Spinnpräparationen für synthetische Fasern, insbesondere Acryl- und Polyesterfasern, enthaltend quaternierte Fettsäuretriethanolaminester-Salze der Formel (I), 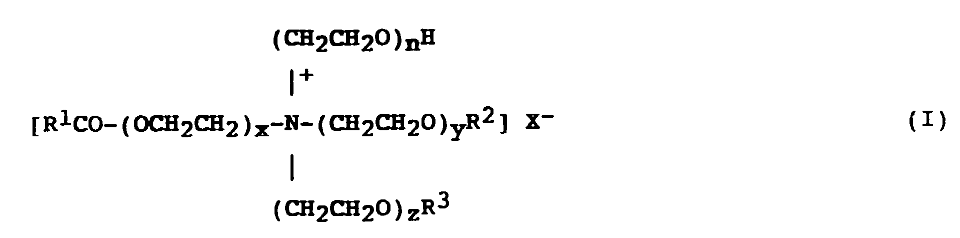 in der R¹CO für einen Acylrest mit 6 bis 22 Kohlenstoffatomen, R² und R³ unabhängig voneinander für Wasserstoff oder R¹CO, n für Zahlen von 1 bis 10, x, y und z für 1 oder Zahlen von 2 bis 30 und X für ein langkettiges Alkyl- und/oder Dialkylphosphat steht.

Wie in der Textilindustrie üblich, werden die Spinnpräparationen in Form wäßriger Dispersionen auf die synthetischen Fasern unmittelbar nach Austritt aus der Spinndüse appliziert. Die Spinnpräparationen, die eine Temperatur zwischen 18 und 90°C haben, werden z. B. von Auftragwalzen oder Dosierpumpen über geeignete Applikatoren aufgebracht. Bevorzugt werden Spinnpräparationen in Form ihrer wäßrigen Dispersionen eingesetzt, die einen Feststoffgehalt von 3 bis 40, vorzugsweise 5 bis 30 Gew.-% aufweisen.

Die Auftragmenge der Spinnpräparationen in Form ihrer wäßrigen Dispersionen liegt in dem für die Textilindustrie üblichen Bereich zwischen 0,1 und 3 Gew.-% - bezogen auf das Gewicht der Filamentfasern.

Ein weiterer Gegenstand der Erfindung betrifft schließlich die Verwendung von quaternierten Fettsäuretriethanolaminester-Salzen für die Herstellung von Spinnpräparationen für synthetische Fasern.

Die folgenden Beispiele sollen den Gegenstand der Erfindung näher erläutern, ohne ihn darauf einzuschränken.

### Beispiele

### I. Allgemeine Herstellungsvorschrift

a) **Veresterung.** In einem 1-1-Dreihalskolben mit Rührer, Innenthermometer und Destillationsaufsatz wurden 1,9 mol Fettsäure, 1 mol Triethanolamin und 1,4 g 50 gew.-%ige unterphosphorige Säure gegeben. Über einen Zeitraum von 4 h wurde die Reaktionsmischung bei einem verminderten Druck von 40 mbar auf eine Temperatur von 160°C erhitzt, bis die Säurezahl unterhalb von 5 lag. Anschließend wurde der rohe Fettsäure-triethanolaminester abgekühlt, der Reaktionsansatz entspannt und unter ständigem Rühren innerhalb von 15 min 1 Liter Luft durchgeleitet.
b) **Quaternierung.** In einem 1-1-Stahlautoklaven wurden 1 mol des technischen Veresterungsproduktes aus a) vorgelegt und mit 1 mol Phosphorsäure-mono/di-ester versetzt. Anschließend wurden innerhalb von 2 h 1 bis 2 Mol Ethylenoxid bei einem Druck von 3 bar und einer Temperatur von 105°C aufgepreßt.

Im Fall Bsp.1 wurde ungehärtete C_{12/18}-Kokosfettsäure, ein Phosphorsäure-octyl/decylester (Mono-/di-Verhältnis = 0,7) und 1 Mol Ethylenoxid eingesetzt.

Im Fall Bsp.2 wurde zu 50 % gehärtete C_{16/18}-Talgfettsäure, Phosphorsäurelaurylester (Mono-/Di-Verhältnis = 0,7) und 1 Mol Ethylenoxid eingesetzt.

Im Fall Bsp.3 wurde zu 50 % gehärtete C_{16/18}-Talgfettsäure, Phosphorsäurelaurylester (Mono-/Di-Verhältnis = 0,7) und 2 Mol Ethylenoxid eingesetzt.

### II. Anwendungsbeispiele

Auf Polyesterfilamente wurde eine 15 gew.-%ige Spinnfaserpräparation des Esterquats gemäß dem Herstellungsbeispiel in Wasser aufgetragen (Ölauflage 0,35 Gew.-%).

Folgende Parameter wurden bestimmt:
- dynamische Reibungskoeffizienten gegen Faden bei einer Geschwindigkeit von 8 mm/min, gemessen am Rothschild F-Meter (Klima 20°C, 65 % relative Feuchte);
- dynamische Reibungskoeffizienten gegen Metall bei einer Geschwindigkeit von 20 bzw. 100 m/min, gemessen am Rothschild F-Meter (Klima 20°C, 65 % relative Feuchte).
- elektrostatische Aufladung an Metall bei einer Geschwindigkeit von 100 m/min, 2 min, T = 20°C, gemessen am Statikvoltmeter Rothschild (Klima 20°C, 65 % rel. Feuchte).
- elektrostatische Aufladung an Metall bei einer Geschwindigkeit von 100 m/min, 2 min, T = 150°C, gemessen am Statikvoltmeter Rothschild (Klima 20°C, 65 % rel. Feuchte).
- Farbstoffausblutung (Farbstoff: Kationisch Schwarz) visuelle Beurteilung:
   I = kein Ausbluten
   II = geringes Ausbluten
   III = starkes Ausbluten
   IV = sehr starkes Ausbluten

**Tab.1:**

| Anwendungsbeispiele | | | | | | |
|---|---|---|---|---|---|---|
| Bsp. | dRK | | | EA1 V | EA2 V | FA |
| | F1 µ | F2 µ | F3 µ | | | |
| 1 | 14 | 0,243 | 0,256 | 0 | 0,5 | I |
| 2 | 15 | 0,189 | 0,230 | 0 | 0,5 | I |
| 3 | 15 | 0,178 | 0,224 | 0 | 0,5 | I |
| Legende: dRK = dynamischer Reibungskoeffizient F1 = dRK gegen Faden (Geschwindigkeit 8 m/min) F2 = dRK gegen Metall (Geschwindigkeit 20 m/min) F3 = dRK gegen Metall (Geschwindigkeit 100 m/min) EA1 = Elektrostatische Aufladung ( 20°C) EA2 = Elektrostatische Aufladung (150°C) FA = Farbstoffausblutung | | | | | | |

## Patentansprüche

1. Quaternierte Fettsäure-triethanolaminester-Salze der Formel **(I)**, 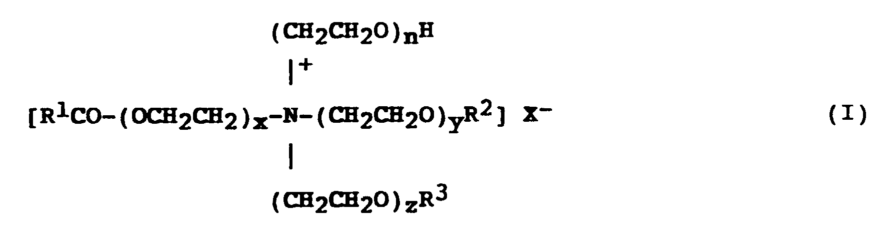 in der R¹CO für einen Acylrest mit 6 bis 22 Kohlenstoffatomen, R² und R³ unabhängig voneinander für Wasserstoff oder R¹CO, n für Zahlen von 1 bis 10, x, y und z für 1 oder Zahlen von 2 bis 30 und X für ein langkettiges Alkyl- und/oder Dialkylphosphat steht.

2. Verfahren zur Herstellung von quaternierten Fettsäuretriethanolaminester-Salzen der Formel **(I)**, 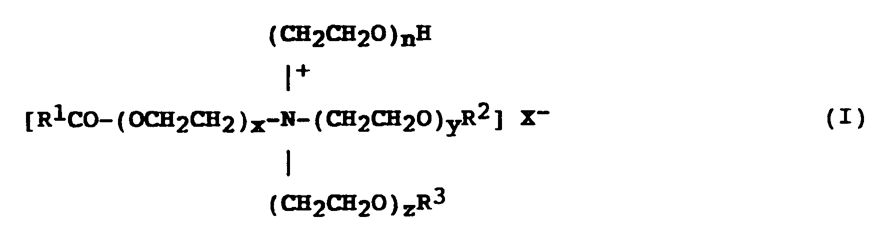 in der
R¹CO für einen Acylrest mit 6 bis 22 Kohlenstoffatomen, R² und R³ unabhängig voneinander für Wasserstoff oder R¹CO, n für Zahlen von 1 bis 10, x, y und z für 1 oder Zahlen von 2 bis 30 und X für ein langkettiges Alkyl- und/ oder Dialkylphosphat steht, bei dem man Fettsäuren mit Triethanolamin oder Triethanolaminethoxylaten verestert und anschließend in Gegenwart von langkettigen Phosphorsäure-mono/di-estern mit Ethylenoxid quaterniert.

3. Verfahren nach Anspruch 2, **dadurch gekennzeichnet**, daß man Fettsäuren der Formel **(II)** einsetzt,
**R**^{**4**}**CO-OH (II)**
in der R⁴CO für einen aliphatischen, gesättigten oder ungesättigten Acylrest mit 6 bis 22 Kohlenstoffatomen steht.

4. Verfahren nach den Ansprüchen 2 und 3, **dadurch gekennzeichnet**, daß man Triethanolamin bzw. Triethanolaminethoxylate der Formel **(III)** einsetzt, 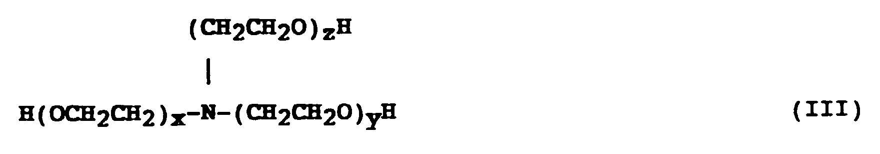 in der x, y und z für 1 oder Zahlen von 2 bis 30 stehen können.

5. Verfahren nach den Ansprüchen 2 bis 4, **dadurch gekennzeichnet**, daß man die Fettsäuren und das Triethanolamin bzw. Triethanolaminethoxylat im molaren Verhältnis von 1 : 1,0 bis 1 : 3,0 einsetzt.

6. Verfahren nach den Ansprüchen 2 bis 5, **dadurch gekennzeichnet**, daß man die Veresterung bei Temperaturen von 120 bis 220°C und Drücken von 0,01 bis 1 bar durchführt.

7. Verfahren nach den Ansprüchen 2 bis 6, **dadurch gekennzeichnet**, daß man die Quaternierung in Gegenwart von langkettigen Phosphorsäuremono/di-estern der Formel (IV) durchführt, 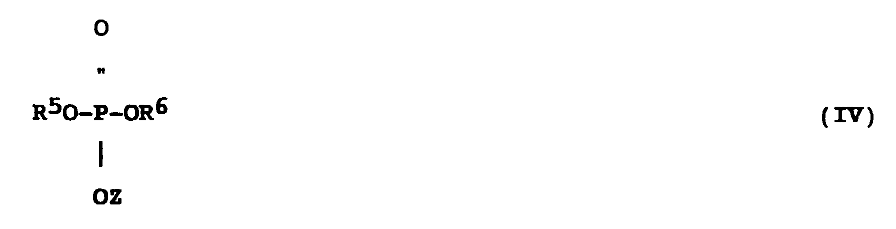 in der R⁵ für einen Alkyl- und/oder Alkenylrest mit 4 bis 18 Kohlenstoffatomen oder einen Rest R⁷(OCH₂CH₂)ₘ, R⁶ für Wasserstoff, einen Alkyl- und/oder Alkenylrest mit 4 bis 18 Kohlenstoffatomen oder einen Rest H(OCH₂CH₂)ₘ, R⁷ für einen Alkyl- und/oder Alkenylrest mit 4 bis 18 Kohlenstoffatomen, m für Zahlen von 1 bis 8 und Z für Wasserstoff oder ein Alkalimetall steht.

8. Verfahren nach den Ansprüchen 2 bis 7, **dadurch gekennzeichnet**, daß man die Veresterungsprodukte von Fettsäure und Triethanolamin und die Phosphorsäureester im molaren Verhältnis von 1 : 0,5 bis 1 : 1,5 einsetzt.

9. Verfahren nach den Ansprüchen 2 bis 8, **dadurch gekennzeichnet**, daß man die Veresterungsprodukte von Fettsäure und Triethanolamin bzw. Triethanolaminethoxylat und das Ethylenoxid im molaren Verhältnis von 1 : 1 bis 1 : 10 einsetzt.

10. Verfahren nach den Ansprüchen 2 bis 9, **dadurch gekennzeichnet**, daß man die Quaternierung bei Temperaturen von 50 bis 150°C und 1 bis 10 bar durchführt.

11. Spinnpräparationen für synthetische Fasern, enthaltend quaternierte Fettsäure-triethanolaminester-Salze der Formel **(I)**, 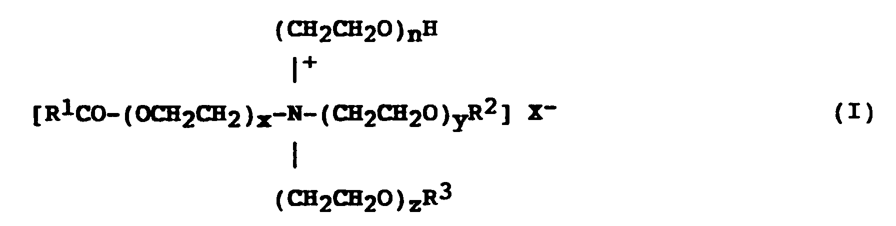 in der R¹CO für einen Acylrest mit 6 bis 22 Kohlenstoffatomen, R² und R³ unabhängig voneinander für Wasserstoff oder R¹CO, n für Zahlen von 1 bis 10, x, y und z für 1 oder Zahlen von 2 bis 30 und X für ein langkettiges Alkyl- und/oder Dialkylphosphat steht.

12. Verwendung von quaternierten Fettsäure-triethanolaminester-Salzen nach Anspruch 1 für die Herstellung von Spinnpräparationen für synthetische Fasern.

## Claims

1. Quaternized fatty acid triethanolamine ester salts corresponding to formula **(I)**: 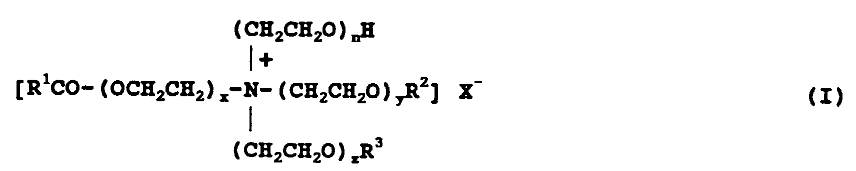 in which R¹CO is an acyl group containing 6 to 22 carbon atoms, R² and R³ independently of one another represent hydrogen or R¹CO, n is a number of 1 to 10, x, y and z = 1 or numbers of 2 to 30 and X is a long-chain alkyl and/or dialkyl phosphate.

2. A process for the production of quaternized fatty acid triethanolamine ester salts corresponding to formula **(I)**: 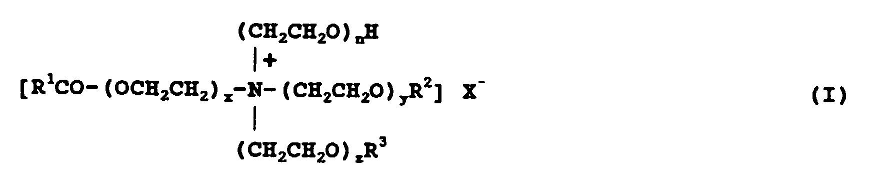 in which R¹CO is an acyl group containing 6 to 22 carbon atoms, R² and R³ independently of one another represent hydrogen or R¹CO, n is a number of 1 to 10, x, y and z = 1 or numbers of 2 to 30 and X is a long-chain alkyl and/or dialkyl phosphate,
**characterized in that** fatty acids are esterified with triethanolamine or triethanolamine ethoxylates and subsequently quaternized with ethylene oxide in the presence of long-chain phosphoric acid mono-/diesters.

3. A process as claimed in claim 2, **characterized in that** fatty acids corresponding to formula (II):
**R**^{**4**}**CO-OH (II)**
in which R⁴CO is an aliphatic, saturated or unsaturated acyl group containing 6 to 22 carbon atoms, are used.

4. A process as claimed in claims 2 and 3, **characterized in that** triethanolamine or triethanolamine ethoxylates corresponding to formula **(III)**: 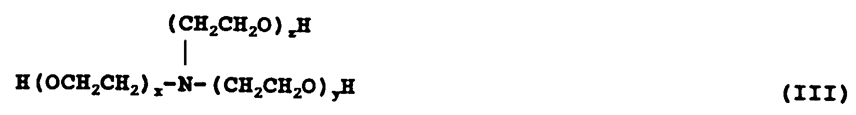 in which x, y and z = 1 or numbers of 2 to 30,
are used

5. A process as claimed in claims 2 to 4, **characterized in that** the fatty acids and the triethanolamine or triethanolamine ethoxylate are used in a molar ratio of 1:1.0 to 1:3.0.

6. A process as claimed in claims 2 to 5, **characterized in that** the esterification is carried out at temperatures of 120 to 220°C under pressures of 0.01 to 1 bar.

7. A process as claimed in claims 2 to 6, **characterized in that** the quaternization is carried out in the presence of long-chain phosphoric acid mono-/diesters corresponding to formula **(IV)**: 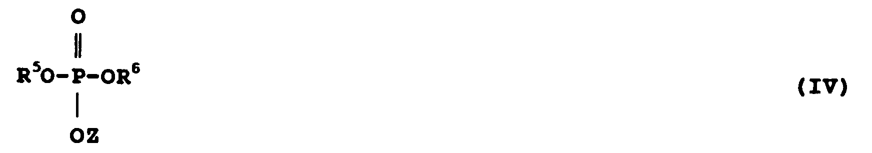 in which R⁵ is an alkyl and/or alkenyl group containing 4 to 18 carbon atoms or a group R⁷(OCH₂CH₂)ₘ, R⁶ is hydrogen, an alkyl and/or alkenyl group containing 4 to 18 carbon atoms or a group H(OCH₂CH₂)ₘ, R⁷ is an alkyl and/or alkenyl group containing 4 to 18 carbon atoms, m is a number of 1 to 8 and Z is hydrogen or an alkali metal.

8. A process as claimed in claims 2 to 7, **characterized** **in that** the esterification products of fatty acid and triethanolamine and the phosphoric acid esters are used in a molar ratio of 1:0.5 to 1:1.5.

9. A process as claimed in claims 2 to 8, **characterized in that** the esterification products of fatty acid and triethanolamine or triethanolamine ethoxylate and the ethylene oxide are used in a molar ratio of 1:1 to 1:10.

10. A process as claimed in claims 2 to 9, **characterized in that** the quaternization is carried out at temperatures of 50 to 150°C under pressures of 1 to 10 bar.

11. Spin finishes for synthetic fibers containing quaternized fatty acid triethanolamine ester salts corresponding to formula **(I)**: 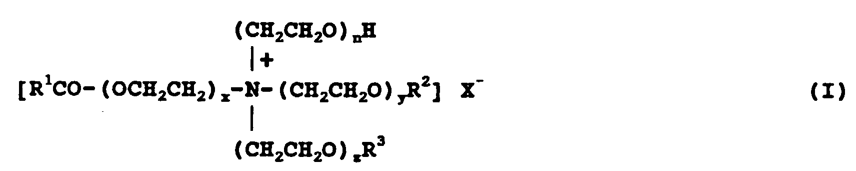 in which R¹CO is an acyl group containing 6 to 22 carbon atoms, R² and R³ independently of one another represent hydrogen or R¹CO, n is a number of 1 to 10, x, y and z = 1 or numbers of 2 to 30 and X is a long-chain alkyl and/or dialkyl phosphate.

12. The use of the quaternized fatty acid triethanolamine ester salts claimed in claim 1 for the production of spin finishes for synthetic fibers.

## Revendications

1. Sels quaternaires d'esters de triéthanolamine d'acide gras de la formule (I), 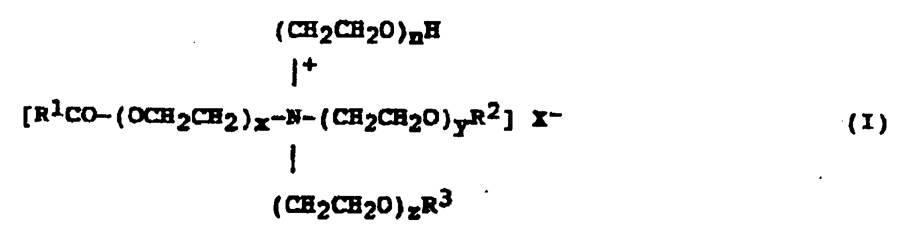 dans laquelle R¹CO représente un radical acyle comportant 6 à 22 atomes de carbone, R² et R³ correspondent indépendamment l'un de l'autre à l'hydrogène ou à R¹CO, n représente un nombre de 1 à 10, x, y et z équivalent, chacun, à 1 ou à un nombre de 2 à 30, et X correspond à un alkyl- et/ou à un dialkylphosphate à chaîne longue.

2. Procédé de fabrication d'esters de triéthanolamine d'acide gras de la formule (I), 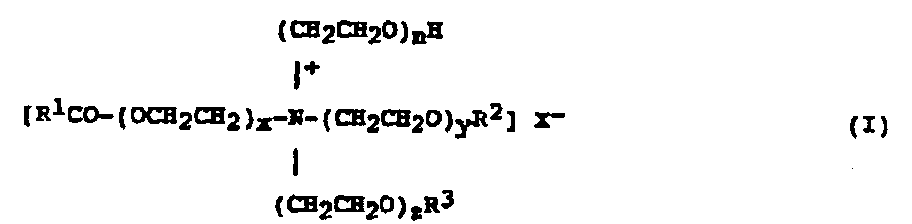 dans laquelle R¹CO représente un radical acyle comportant 6 à 22 atomes de carbone, R² et R³ correspondent indépendamment l'un de l'autre à l'hydrogène ou à R¹CO, n représente un nombre de 1 à 10, x, y et z équivalent, chacun, à 1 ou à un nombre de 2 à 30, et X correspond à un alkyl- et/ou à un dialkylphosphate à chaîne longue, dans lequel on estérifie des acides gras avec de la triéthanolamine ou avec des éthoxylates de triéthanolamine, et on les quaternise ensuite avec de l'oxyde d'éthylène, en présence de mono-/diesters d'acide phosphorique à chaîne longue.

3. Procédé selon la revendication 2, caractérisé en ce que l'on utilise des acides gras de la formule (II),
R⁴CO-OH (II)
dans laquelle R⁴CO représente un radical acyle aliphatique, saturé ou insaturé, comportant 6 à 22 atomes de carbone.

4. Procédé selon les revendications 2 et 3, caractérisé en ce que l'on utilise de la triéthanolamine ou des éthoxylates de triéthanolamine de la formule (III), 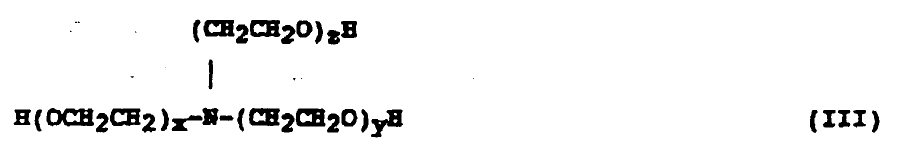 dans laquelle x, y et z peuvent représenter, chacun, 1 ou un nombre de 2 à 30.

5. Procédé selon les revendications 2 à 4, caractérisé en ce que l'on met en oeuvre les acides gras et la triéthanolamine ou l'éthoxylate de triéthanolamine dans un rapport molaire de 1:1,0 à 1:3,0.

6. Procédé selon les revendications 2 à 5, caractérisé en ce que l'on opère l'estérification à des températures de 120 à 220 °C et à des pressions de 0,01 à 1 bar.

7. Procédé selon les revendications 2 à 6, caractérisé en ce que l'on opère la quaternisation en présence de mono/diesters d'acide phosphorique à chaine longue de la formule (IV), 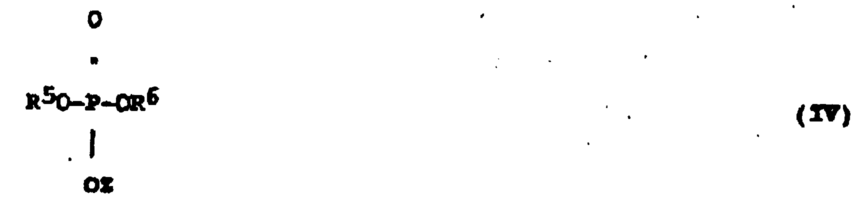 dans laquelle R⁵ représente un radical alkyle et/ou alcényle comportant 4 à 18 atomes de carbone ou un radical R⁷(OCH₂CH₂)ₘ, R⁶ correspond à l'hydrogène, à un radical alkyle et/ou alcényle comportant 4 à 18 atomes de carbone ou à un radical H(OCH₂CH₂)ₘ, R⁷ représente un radical alkyle et/ou alcényle comportant 4 à 18 atomes de carbone, m équivaut à un nombre de 1 à 8 et Z représente l'hydrogène ou un métal alcalin.

8. Procédé selon les revendications 2 à 7, caractérisé en ce que les produits d'estérification de l'acide gras et de la triéthanolamine et les esters d'acide phosphorique sont mis en oeuvre dans un rapport molaire de 1:0,5 à 1:1,5.

9. Procédé selon les revendications 2 à 8, caractérisé en ce que les produits d'estérification de l'acide gras et de la triéthanolamine ou de l'éthoxylate de triéthanolamine et l'oxyde d'éthylène sont mis en oeuvre dans un rapport molaire de 1:1 à 1:10.

10. Procédé selon les revendications 2 à 9, caractérisé en ce que l'on opère la quaternisation à une température de 50 à 150 °C et à une pression de 1 à 10 bars.

11. Préparations de filature pour les fibres synthétiques contenant des sels quaternaires d'esters de triéthanolamine d'acide gras de la formule (I), 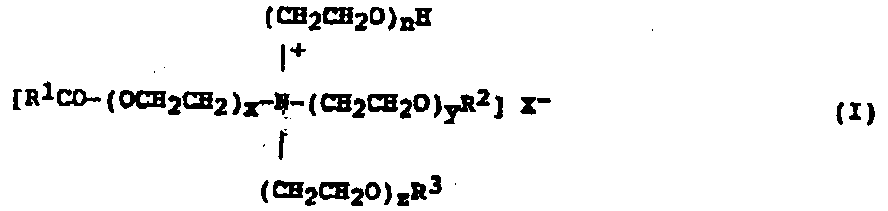 dans laquelle R¹CO représente un radical acyle comportant 6 à 22 atomes de carbone, R² et R³ correspondent indépendamment l'un de l'autre à l'hydrogène ou à R¹CO, n représente un nombre de 1 à 10, x, y et z équivalent, chacun, à 1 ou à un nombre de 2 à 30, et X correspond à un alkyl- et/ou à un dialkylphosphate à chaîne longue.

12. Utilisation de sels quaternaires d'esters de triéthanolamine d'acide gras selon la revendication 1 pour la fabrication de préparations de filature pour les fibres synthétiques.
